# EUROPÄISCHE PATENTSCHRIFT

(11) **EP 3 950 252 B1**
(45) Veröffentlichungstag und Bekanntmachung des Hinweises auf die Patenterteilung: **13.03.2024**
(21) Anmeldenummer: 20189633.9
(22) Anmeldetag: 05.08.2020
(51) Int. Cl.: B29B 17/04, C08C 19/08, C08J 11/10, C12R 1/01

(54) **VERFAHREN ZUR DEVULKANISATION VON GUMMIPARTIKELN**
PROCESS FOR DEVULCANISATION OF RUBBER PARTICLES
PROCÉDÉ DE DEVULCANISATION DE PARTICULES D'CAOUTCHOUC

(43) Veröffentlichungstag der Anmeldung: 09.02.2022
(73) Patentinhaber: RubberSubstitute4.0 SA, 1195 Dully (CH)
(72) Erfinder:
(74) Vertreter: König Szynka Tilmann von Renesse Patentanwälte Partnerschaft mbB Düsseldorf

(56) Entgegenhaltungen:
- EP-A2- 0 821 982
- WO-A1-2004/087799
- WO-A1-2016/008950
- WO-A2-01/74933
- US-A- 5 344 778
- US-A1- 2011 301 302
- US-A1- 2014 178 970
- US-A1- 2017 349 723
- Mohsen Sohrabi ET AL: "Bacterial Desulfurization of Organic Sulfur Compounds Exist in Fossil Fuels", , 15. Juli 2011 (2011-07-15), XP055738126, Gefunden im Internet: URL:https://www.researchgate.net/profile/H esam_Kamyab4/publication/262535818_Bacteri al_Desulfurization_of_Organic_Sulfur_Compo unds_Exist_in_Fossil_Fuels/links/0c960537f 2d804b910000000/Bacterial-Desulfurization- of-Organic-Sulfur-Compounds-Exist-in-Fossi l-Fuels.pdf [gefunden am 2020-10-08]
- ERICH MARKL ET AL: "Devulcanization Technologies for Recycling of Tire-Derived Rubber: A Review", MATERIALS, Bd. 13, Nr. 5, 10. März 2020 (2020-03-10), Seite 1246, XP055738140, CH ISSN: 1996-1944, DOI: 10.3390/ma13051246

## Beschreibung

### Technisches Gebiet der Erfindung

Die vorliegende Erfindung betrifft ein Verfahren zur Devulkanisation von Gummipartikeln, umfassend die Kultivierung von Bakterien der Gattung *Sporoanaerobacter, Desulfovibrio, Desulfurella* und/oder *Desulfomicrobium* mit den Gummipartikeln.

Weiterhin betrifft die Erfindung eine Mischpopulation, umfassend Bakterien der Gattung *Sporoanaerobacter, Desulfovibrio, Desulfurella* und/oder *Desulfomicrobium.*

Die vorliegende Erfindung betrifft auch die Verwendung der Mischpopulation zur Wiederverwertung von Altgummi.

### Hintergrund der Erfindung/Stand der Technik

Die werkstoffliche Wiederverwertung zerkleinerter schwefelvernetzter Altgummierzeugnisse (schwefelvernetzter Synthesekautschuke und Naturkautschuke) ist aus wirtschaftlicher und ökologischer Sicht eine zwingende Notwendigkeit. Anzustreben ist dabei die Rückführung der behandelten Altgummierzeugnisse in den Gummikreislauf.

Obwohl weltweit an der Entwicklung entsprechender Verfahren gearbeitet wird, hat sich gegenwärtig noch kein Verfahren durchgesetzt.

Hauptursache sind mangelhafte Produktqualitäten, unzureichende Wirtschaftlichkeiten und Kapazitätsbegrenzungen der Verfahren sowie spezifische technisch-technologische Schwierigkeiten, die hauptsächlich auf die Vielfalt an unterschiedlichen Gummitypen in Gummierzeugnissen zurückzuführen sind. In einem Übersichtsartikel von Sohrabi und anderen im "Journal of Pure and Applied Microbiology (2012) 6(2), Seite 717-729" sind zahlreiche Beispiele von Entschwefelungen schwefelorganischer Verbindungen mittels Bakterien beschrieben.

### Mechanische und chemische Verfahren

Bei der mechanischen Aktivierung wird das Gummimehl/Gummigranulat durch speziell strukturierte Walzen, Extruder oder die mechanische Einwirkung anderer Maschinen destrukturiert, was zu einer Vergrößerung der spezifischen Oberfläche des Gummimehls und dem partiellen Bruch der Schwefelbrücken führt.

Die technische Anwendung dieser Verfahren hat in der Regel gezeigt, dass die Aktivierungswirkung bei der Verarbeitung des Gummimehls in Innenmischern, Extrudern, Walzen usw. durch die mechanische Belastung und die erhöhten Temperaturen verloren geht.

Die entwickelten chemischen und mechanischen Aktivierungsverfahren sind zu teuer, zu arbeitsintensiv und auch aus Gründen des Arbeits- und Umweltschutzes abzulehnen. Einige chemische Verfahren bewirken zudem eine schädigende partielle Depolymerisation (Abbau der Kautschukmoleküle).

Gleichfalls nicht durchgesetzt haben sich Devulkanisationsverfahren, die auf der Einwirkung von Ultraschallenergie auf das Gummimehl beruhen.

In DE 4425049 C1, DE 19607281 A1 und US 5,506,283 sind unterschiedliche Aktivierungsverfahren von Abfallgummi und zerkleinertem Altgummi beschrieben, die auf chemischen und/oder physikalischen Wirkprinzipien beruhen.

### Mikrobielle Schwefeloxidation

In den letzten Jahren sind verstärkt Verfahren vorgeschlagen bzw. entwickelt worden, die auf einer mikrobiellen und/oder enzymatischen Aktivierung von schwefelvernetzten Gummimehlen beruhen. Die Oxidation des Schwefels erfolgt dabei in der Regel bis zum Sulfat.

Unterschiedliche Verfahren sind unter anderem in folgenden Dokumenten beschrieben: DE 4042009 C2, DE 2724813 A1, GB 2097817 A, EP 0493732 B1, EP 402704 A1, US 5,597,851, DE 19728036 A1, US 6,479,558 B1, US 6,217,766 B1 und WO 2016/008950.

Obwohl die Möglichkeit der Aktivierung von schwefelvernetzten Gummimehlen durch mikrobielle Oxidation des polysulfidisch gebundenen Schwefels überzeugend nachgewiesen wurde, weisen die bisher vorgeschlagenen Verfahren eine Reihe signifikanter Nachteile auf:
Unter großtechnischen Bedingungen wird der Prozess zwangsläufig unter unsterilen Bedingungen betrieben, sodass durch den Wasser- und Lufteintrag Fremdorganismen in die Bioreaktoren transportiert werden. Diese Fremdorganismen sind vielfältiger Natur. Einige konkurrieren mit den Schwefeloxidierern lediglich um bestimmte Nährstoffe, leben aber sonst im Neutralismus. Andere, z.B. Ciliaten und Flagellaten (in diesem Falle Parasiten) vernichten zudem noch Schwefeloxidierer.

Während des Oxidationsprozesses werden bestimmte biotoxische Stoffe aus dem Gummimehl in die flüssige Phase des Bioreaktors transportiert (Alterungsschutzmittel, Zinkoxid usw.), was zu einer Hemmung bzw. Einstellung des Stoffwechsels und des Wachstums der Mikroorganismen führt. Da die Resistenz der bekannten Schwefeloxidierer gegen die oben genannten biotoxischen Stoffe relativ gering ist, muss die Gummimehlkonzentration in den Bioreaktoren auf einem niedrigen Niveau gehalten werden, was die Wirtschaftlichkeit des Verfahrens stark reduziert.

Bei der Verwendung thermophiler Mikroorganismen für die oxidative (aerobe) Gummimehlentschwefelung (z.B. *Sulfolobus acidocaldarius*) verläuft der Entschweflungsprozess relativ intensiv. Durch die verstärkte Freisetzung unterschiedlicher Gummiinhaltsstoffe (Ruß, Weichmacher, Zinkoxid, Alterungsschutzmittel usw.) kommt es allerdings zu einer nicht akzeptablen Schädigung der Elastomermatrix sowie zu einer hohen Belastung des Prozessabwassers mit toxischen Stoffen. Die relativ hohen Reaktionstemperaturen wirken sich zudem negativ auf die Wirtschaftlichkeit des Verfahrens aus.

### Mikrobielle Schwefelreduktion

Um die Nachteile von Verfahren zur Entschwefelung von Gummimehl mit aeroben Bakterien zu vermeiden, werden anaerobe Entschwefelungsverfahren getestet, bei denen der Schwefel zu Schwefelwasserstoff reduziert wird.

K. Bredberg und andere beschreiben in "Appl. Microbiol. Biotechnol (2001) 55, Seite 43-48" ein Entschwefelungsverfahren für Gummi unter Verwendung des extrem thermophilen Archaeons *Pyrococcues furiosus.* Das Temperaturoptimum dieses Bakteriums liegt bei 90 - 100 °C. Die Nachteile dieses Verfahrens sind allerdings identisch mit den Nachteilen thermophiler aerober Entschwefelungsverfahren: signifikante Schädigung der Elastomermatrix und der Mikroorganismenpopulation durch die intensive Freisetzung von Gummiinhaltsstoffen sowie starke toxische Belastung des Prozessabwassers.

In WO 2004/087799 A1 wird ein anaerobes Verfahren beschrieben, in dem mit den folgenden mesophilen Bakterien positive Resultate bei der Gummimehlentschwefelung erzielt wurden: *Desulfuromonas thiophila, Desulfuromonas palmitatis, Sulfurospirillum deleyianum, Desulfuromonas acetoxidans.*

Diese Bakterien sind als Sulfatreduzierer bekannt.

Untersuchungen über einen längeren Zeitraum haben gezeigt, dass diese Bakterien prinzipiell in der Lage sind, auch den polysulfidisch gebundenen Schwefel an der Oberfläche zu reduzieren. Sie weisen aber eine Reihe fundamentaler Nachteile auf, die einen Einsatz unter großtechnischen Bedingungen praktisch ausschließen. Dazu gehören Schwierigkeiten bei der Kultivierung/Vermehrung in Aufzuchtbioreaktoren,

Bedarf an teuren Nährstoffen, zu geringe Resistenz gegen toxische Inhaltsstoffe der Gummimehle (Alterungsschutzmittel, Zinkoxid usw.) und gegen den gebildeten Schwefelwasserstoff sowie teilweise Enzymfixierung in den Zellmembranen.

In der US 5 344 778 A wird beschrieben, dass Extrakte, die Membranfragmente und/oder Enzyme von *Rhodococcus rhodochrous* und *Bacillus sphaericus* umfassen, die Fähigkeit aufweisen, den Schwefelgehalt von schwefelhaltigem organischem Material selektiv zu reduzieren. Aus der US2017349723 ist eine ein Desulfomicrobium umfassende Mischpopulation von Bakterien (als Zuschlagstoff für einen Biologisch abbaubaren Kunststoff) bekannt, während aus der US2014178970 Mischpopulationen umfassend Desulfomicrobium bzw. Desulfovibrio (zur Aufbereitung langer Kohlenwasserstoffe) bekannt sind.

Eine Aufgabe der vorliegenden Erfindung ist somit, ein Verfahren bereitzustellen, das Nachteile des Standes der Technik überwindet und besser als bisherige Verfahren zur Devulkanisation von Gummi eingesetzt werden kann.

### Kurzbeschreibung der Erfindung

Erfindungsgemäß wurde überraschend gefunden, dass Gummipartikel mittels Bakterien der Gattungen *Sporoanaerobacter, Desulfovibrio, Desulfurella* und/oder *Desulfomicrobium* effizient devulkanisiert werden können. Dieses neuartige Verfahren erlaubt es beispielsweise Altgummi in besonders wirtschaftlicher und zugleich umweltschonender Weise wiederzuverwerten.

Die vorliegende Erfindung betrifft dabei die folgenden Aspekte:
(1) ein Verfahren zur Devulkanisation von Gummipartikeln, umfassend die Kultivierung von Bakterien der Gattung *Sporoanaerobacter, Desulfovibrio, Desulfurella* und/oder *Desulfomicrobium* mit Gummipartikeln;
(2) eine Mischpopulation, umfassend Bakterien der Gattung *Sporoanaerobacter, Desulfovibrio, Desulfurella* und *Desulfomicrobium;*
(3) die Verwendung der Mischpopulation zur Wiederverwertung von Altgummi. Weitere, bevorzugte Aspekte der Erfindung sind in den abhängigen Ansprüchen beschrieben.

### Kurzbeschreibung der Figuren

Figur 1: Vergleich der Vulkanisationsisothermen verschiedener Gummimischungen; "ungefüllt": Standardgummimischung ohne Gummimehlzusatz;
   "GM1": Standardgummimischung mit unbehandeltem Gummimehl;
   "GM2": Standardgummimischung mit devulkanisiertem Gummimehl.
Figur 2: Vergleich der Vulkanisationsisothermen (bei einer Temperatur von 170 °C) synthetischer Kautschukmischungen [Ethylen-Propylen-Dien (EPDM)-Mischungen]; "ungefüllt": EPDM-Mischung ohne Gummimehlzusatz;
   "GM A, 31 phr, unbehandelt": EPDM Mischung mit 31 parts per hundred rubber (phr) unbehandeltem Gummimehl;
   "GM A, 75 phr, unbehandelt": EPDM Mischung mit 75 phr unbehandeltem Gummimehl;
   "GM B, 31 phr", behandelt: EPDM Mischung mit 31 phr devulkanisiertem Gummimehl;
   "GM B, 75 phr, behandelt", EPDM Mischung mit 75 phr devulkanisiertem Gummimehl.

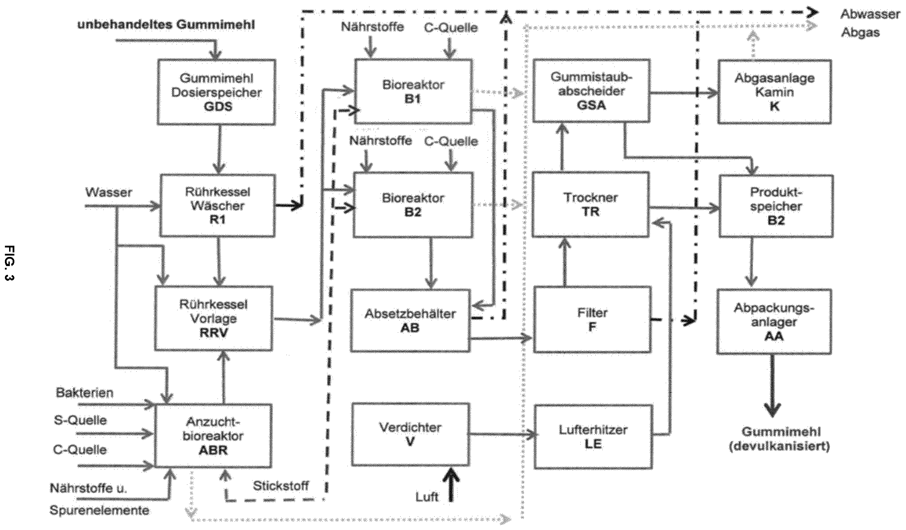

Figur 3: Vereinfachtes technologisches Verfahrensschema einer kleintechnischen mikrobiologischen Oberflächenentschwefelung mit den folgenden Prozessstufen (verfahrenstechnischen Grundoperationen): Lagerung/Bevorratung von Gummimehl; Waschung (Hydrophilierung, Entgiftung) des Gummimehls; Entsorgung des kontaminierten Prozesswassers als Abwasser nach Abtrennung des gewaschenen Gummimehls; Bereitstellung, Lagerung und Proportionierung der erforderlichen Stoffkomponenten (Mikroorganismen, Schwefelsubstrate, Kohlenstoffquellen, Nährstoffe, Vitamine, Spurenelemente usw.); Mikroorganismenanzucht/-vermehrung; Gummimehlentschwefelung; Behandlung und Entsorgung der entstehenden Abgase; Gummimehlabtrennung von der flüssigen Phase (Sedimentation, Filtration); Entsorgung des entstehenden Abwassers; Trocknung des behandelten Gummimehls; Verpackung des getrockneten und entstaubten Gummimehls.

### Detaillierte Beschreibung der Erfindung

Die vorliegende Erfindung betrifft das oben unter (1) beschriebene Verfahren zur Devulkanisation von Gummipartikeln. Unter "Devulkanisation" ist im Zusammenhang mit der vorliegenden Erfindung das teilweise und/oder vollständige Aufbrechen von Schwefelbrücken, wie sie insbesondere auf der Oberfläche von Gummipartikeln vorkommen, zu verstehen. Mithin ist unter dem vorliegend beschriebenen Verfahren auch eine Entschwefelung, insbesondere eine Oberflächenentschwefelung zu verstehen.

Grundsätzlich kann die Devulkanisation sowohl oxidativ bzw. unter aeroben Bedingungen als auch reduktiv bzw. unter anaeroben Bedingungen erfolgen. Bei der Devulkanisation entsprechend der vorliegenden Erfindung handelt es sich bevorzugt um eine Reduktion bzw. eine Devulkanisation unter anaeroben Bedingungen. Durch das Fehlen des Lufteintrages bei einem solchen anaeroben Verfahren werden wesentlich weniger schädigende Fremdorganismen in die Bioreaktoren eingetragen. Des Weiteren werden ihre Konzentrationen durch die anaeroben Bedingungen und die Art der Nährstoffversorgung der anaeroben Mikroorganismen stark eingeschränkt. In einer besonders bevorzugten Ausführungsform erfolgt die Reduktion des Schwefels bis hin zum Schwefelwasserstoff.

Das erfindungsgemäße Verfahren umfasst die Kultivierung von Bakterien der Gattung *Sporoanaerobacter, Desulfovibrio, Desulfurella* und/oder *Desulfomicrobium.* In einer bevorzugten Ausführungsform umfasst das Verfahren die Kultivierung einer oder mehrerer der folgenden Bakterienarten: *Sporanaerobacter acetigenes, Desulfovibrio vulgaris, Desulfurella amilsii* und *Desulfomicrobium baculatum.*

Ganz besonders bevorzugt ist es, langzeitlich an die biotoxischen Inhaltsstoffe der Gummimehle adaptierte Bakterien einzusetzen. Dies ermöglicht es, mit höheren, z.B. um ca. 10 % höheren Gummimehlkonzentrationen in den Bioreaktoren zu arbeiten und damit eine Verbesserung der Wirtschaftlichkeit des Devulkanisationsverfahrens zu erreichen. Insbesondere kann durch den Einsatz besonders aktiver und resistenter Schwefelreduzierer eine Verkürzung der Devulkanisationszeit um ca. 15 % erreicht werden.

In einer Ausführungsform der vorliegenden Erfindung umfasst das Verfahren zur Devulkanisation von Gummipartikeln eine Vorkultur sowie eine Hauptkultur. Die Vorkultur dient dabei der Vermehrung schwefelreduzierender Bakterien sowie deren Anpassung an biotoxische Gummiinhaltsstoffe, wie z.B. Alterungsschutzmittel, Vulkanisationsbeschleuniger, Zinkoxid, Ruß, Weichmacher usw. Für die Vorkultur werden bevorzugt Bakterien der Gattung *Sporoanaerobacter, Desulfovibrio, Desulfurella* und/oder *Desulfomicrobium* eingesetzt. Weiter bevorzugt wird für die Vorkultur eine Mischpopulation von *Sporanaerobacter acetigenes, Desulfovibrio vulgaris, Desulfurella amilsii* und *Desulfomicrobium baculatum* eingesetzt. Besonders bevorzugt werden die jeweiligen Bakterienarten in der Vorkultur initial zu gleichen Anteilen eingesetzt. Somit umfasst eine ganz besonders bevorzugte Ausführungsform des erfindungsgemäßen Verfahrens eine Vorkultur, deren initiales Inokulum gleiche Anteile von *Sporanaerobacter acetigenes, Desulfovibrio vulgaris, Desulfurella amilsii* und *Desulfomicrobium baculatum* enthält.

Während der Vorkultur erfolgt eine Vermehrung der Bakterien unter Verwendung von leicht reduzierbaren Schwefelsubstraten wie z.B. Schwefelpulver oder Natriumthiosulfat. Es ist bevorzugt, dass die Vorkultur andauert bis das vorgelegte Schwefelsubstrat vollständig verbraucht (reduziert) ist. Erkennbar ist dieser Zustand, und damit das bevorzugte Ende der Vorkultur, daran, dass die Schwefelwasserstoffbildung eingestellt wird, was sich analytisch verfolgen lässt.

Um außerdem eine gewisse Adaptation der Bakterien an biotoxische Gummiinhaltsstoffe, wie z.B. Alterungsschutzmittel, Vulkanisationsbeschleuniger, Zinkoxid, Ruß, Weichmacher usw., zu gewährleisten, kann die Vorkultur mit geringen Mengen an Gummimehl/Gummipartikeln, z.B. etwa 2 % (w/w), beaufschlagt werden.

In einer Ausführungsform der vorliegenden Erfindung umfasst das Verfahren die oben beschriebene Vorkultur und eine sich daran anschließende Hauptkultur. Für die Hauptkultur wird die Vorkultur, nach Vermehrung und Adaptation wie oben beschrieben, in einen mit Gummimehl/Gummipartikeln beladenen Bioreaktor geleitet. In den Bioreaktor der Hauptkultur wird keine weitere Schwefelquelle (auch nicht in Form von schwefelhaltigen Nährstoffen/Nährsalzen) gegeben. Da der polysulfidische Schwefel auf der Oberfläche des Gummimehls/der Gummipartikel die einzige, verfügbare Schwefelquelle der Hauptkultur darstellt, wird dieser als Substrat reduziert und das Gummimehl/die Gummipartikel so devulkanisiert.

Bevorzugte Prozessparameter für die Hauptkultur umfassen eine Temperatur im Bereich von 30 - 50 °C, einen pH-Wert im Bereich von 4 - 8, eine Dauer von 5 - 10 Tagen sowie eine Gummimehlkonzentration von 15 - 30 %.

Der Vorgang der Devulkanisation ist in der Regel abgeschlossen, sobald die Schwefelwasserstoffbildung zum Erliegen kommt. Dieser Zustand lässt sich leicht analytisch bestimmen. Grundsätzlich ist es durch Messung der Schwefelwasserstoffbildung möglich, die Prozessintensität der Devulkanisation zu bestimmen. Darüber hinaus ist es möglich bzw. zweckmäßig, den Zustand des Entschwefelungsprozesses durch Probenahmen von Gummipartikeln und deren Qualitätsbewertung zu bestimmen (werkstoffliche Testung, Oberflächenbestimmung). Es kann die Freisetzung des biotoxischen Schwefelwasserstoffes durch definierte Zugabe von Eisendreichlorid unterbunden werden (Bildung von Eisensulfid). Letzteres ist zweckmäßig, wenn Gummimehle mit einem sehr hohen Schwefelgehalt verarbeitet werden (Gummimehle aus Hartgummierzeugnissen).

Sowohl während der Vorkultur als auch während der Hauptkultur kann es in Abhängigkeit von den gewählten Prozessbedingungen und hierbei insbesondere in Abhängigkeit von der chemischen Zusammensetzung der Gummimehle/Gummipartikel zu einer Verschiebung bzw. Veränderung der initial eingesetzten Bakterienpopulation kommen. Dieser Umstand wird im Wesentlichen bedingt durch unterschiedliche Resistenzen der einzelnen Bakterienarten im Hinblick auf unterschiedliche biotoxische Inhaltsstoffe bzw. deren Konzentrationen.

Auch ist es weder erforderlich noch unter großtechnischen Bedingungen möglich, mit einer absoluten Reinkultur zu arbeiten. Es kann zudem nicht ausgeschlossen werden, dass einige Bakterien in mutualistischer Symbiose leben. Diese Faktoren tragen weiter dazu bei, dass sich die Mikroorganismenzusammensetzung im Laufe der Zeit verändern kann.

Darüber hinaus können die oben beschriebenen schwefelreduzierenden Bakterienpopulationen auch zusammen mit anderen, beispielsweise fermentativen, acetogenen und/oder methanogenen Bakterien eingesetzt werden, wodurch u.a. Methan, Kohlendioxid, Stickstoff oder andere Gasen freigesetzt werden können. Allerdings ist es bevorzugt, durch Einstellung geeigneter Prozessbedingungen wie z.B. der Substrat- und Nährstoffzusammensetzung, den Anteil nichtschwefelreduzierender Bakterien auf einen unwesentlichen Anteil, z.B. < 5 %, zu reduzieren.

Gasförmiger Stickstoff ist dazu geeignet die Konzentration von biotoxischem Schwefelwasserstoff, der beispielsweise während der Entschwefelung des Gummimehls/der Gummipartikel entsteht, zu reduzieren (Strippung). Entsprechend kann gasförmiger Stickstoff zur Ausstrippung von Schwefelwasserstoff sowie zur Sicherung streng anaerober Verhältnisse in die Vor- und/oder Hauptkulturen geleitet werden.

Sehr vorteilhaft für die Prozessgestaltung hat sich ein Waschen des Gummimehls/der Gummipartikel vor der Devulkanisation erwiesen. Es ist anzunehmen, dass durch eine solche Waschung eine teilweise Entgiftung des Gummimehls/der Gummipartikel erfolgt. Zudem bewirkt das Waschen eine vorteilhafte Hydrophilierung des Gummimehls/der Gummipartikel. Durch das Waschen des Gummimehls/der Gummipartikel und die dadurch erfolgte Konzentrationsreduktion der biotoxischen Giftstoffe kann die Konzentration des Gummimehls/der Gummipartikel im Bioreaktor erhöht werden, ohne dass es zu Prozesseinstellungen kommt. Darüber hinaus wird durch das Waschen die Porosität der Gummipartikel erhöht, u.a. durch die Freisetzung von Alterungsschutzmitteln, Zinkoxid usw., wodurch Enzyme tiefer in das Innere der Gummipartikel eindringen und mehr polysulfidischen Schwefel reduzieren können. Somit umfasst das erfindungsgemäße Verfahren in einer bevorzugten Ausführungsform das Waschen der Gummipartikel vor ihrer Devulkanisation. Insbesondere umfasst das Verfahren das Waschen der Gummipartikel bevor die oben beschriebene Vorkultur in den Bioreaktor der Hauptkultur gegeben wird.

In einer besonders bevorzugten Ausführungsform wird das Gummimehl/die Gummipartikel vor seiner/ihrer Devulkanisation zur Verbesserung seiner/ihrer Benetzbarkeit, zur Oberflächenentgiftung und Oberflächenvergrößerung (Herauslösung von Alterungsschutzmitteln, Zinkoxid usw.) bei 30 - 50 °C in einem Rührkessel 20 - 30 Minuten intensiv mit Wasser gewaschen.

Weitere bevorzugte Prozessparameter betreffen die Durchmischung der verwendeten Bioreaktoren, um eine Sedimentation der Gummipartikel sowie die Bildung verhärteter Gummimehlablagerungen auf dem Reaktorboden zu verhindern.

Des Weiteren ist sowohl eine quasikontinuierliche, bevorzugt aber eine diskontinuierliche Verfahrensführung möglich.

Alternativ zu dem oben beschriebenen mikrobiellen Verfahren kann die Devulkanisation der Gummipartikel zusätzlich oder ausschließlich enzymatisch unter Verwendung entsprechender Enzyme der beschriebenen schwefelreduzierenden Bakterienpopulationen erfolgen.

Das erfindungsgemäße Verfahren ist grundsätzlich zur Devulkanisation einer Vielzahl verschiedener Gummitypen bzw. Gummierzeugnisse, die sich hinsichtlich Korngröße, Korngrößenverteilung, Alter, Zustand, Herkunft und Zerkleinerungsverfahren/- bedingungen unterscheiden können, geeignet.

Aus wirtschaftlichen Gründen und aus Qualitätsgründen werden vornehmlich Gummimehle im Korngrößenbereich kleiner 0,6 mm und größer 0,2 mm verarbeitet (geringe Herstellungskosten der Gummimehle, ausreichende spezifische Oberflächen der Gummipartikeln für eine kovalente chemische Anbindung an Frischgummi).

Die mit dem erfindungsgemäßen Verfahren erhaltenen devulkanisierten Gummipartikel/Gummimehle können beispielsweise zur Produktion von gering belasteten Gummiprodukten aus puren Gummimehlen und/oder zur Produktion von hochbelastbaren Gummiprodukten aus Frischkautschuken mit unterschiedlichen Anteilen zugemischter Gummimehle eingesetzt werden. Es ist bevorzugt, dass die devulkanisierten Gummipartikel/Gummimehle zur Herstellung thermoplastischer Elastomere/TPE-s (z.B. TPE-O, TPE-V) durch Phasenkopplung eingesetzt werden, wobei die Gummipartikel/Gummimehle pur oder in Verbindung mit frischen Ethylen-Propylen-Dien-Mischungen die Weichsegmente/weiche Phase darstellen.

Außerdem können die mit dem erfindungsgemäßen Verfahren erhaltenen devulkanisierten Gummipartikel/Gummimehle zur Herstellung von bestimmten Urethankautschuken verwendet werden, bei denen letztere über Doppelbindungen verfügen und damit mit Schwefel vernetzbar sind.

Die vorliegende Erfindung betrifft auch die oben unter (2) beschriebene Mischpopulation. Unter einer "Mischpopulation" ist im Zusammenhang mit der vorliegenden Erfindung eine bakterielle Zusammensetzung zu verstehen, die Vertreter verschiedener Bakterienstämme, Bakterienarten und/oder Bakteriengattungen enthält.

Eine erfindungsgemäße Mischpopulation umfasst Bakterien der Gattung *Sporoanaerobacter, Desulfovibrio, Desulfurella* und/oder *Desulfomicrobium* In einer bevorzugten Ausführungsform der vorliegenden Erfindung ist die Mischpopulation dadurch erhältlich, dass Bakterien der Gattung *Sporoanaerobacter, Desulfovibrio, Desulfurella* und/oder *Desulfomicrobium* in Anwesenheit von Gummimehl und/oder in Anwesenheit von in Gummimehl vorkommenden biotoxischen Giftstoffen, wie z.B: Alterungsschutzmittel, Vulkanisationsbeschleuniger, Zinkoxid, Ruß, Weichmacher usw., kultiviert werden. Es ist besonders bevorzugt, dass die Mischpopulation dadurch erhältlich ist, dass *Sporanaerobacter acetigenes, Desulfovibrio vulgaris, Desulfurella amilsii* und *Desulfomicrobium baculatum* zu initial gleichen Anteilen in Anwesenheit von Gummimehl/Gummipartikeln kultiviert werden.

Schließlich betrifft die vorliegende Erfindung auch die oben unter (3) beschriebene Verwendung einer Mischpopulation zur Wiederverwertung von Altgummi durch Devulkanisation. Hinsichtlich der zu verwendenden Mischpopulation gelten die Ausführungen zu obigem Aspekt (2) der Erfindung entsprechend. Bevorzugte Ausgestaltungen der Devulkanisation sind im Zusammenhang mit obigem Aspekt (1) der Erfindung beschrieben.

Die Erfindung wird in den nachfolgenden Beispielen näher erläutert.

### Beispiele

### Mikroorganismen

Aus einem Biogasreaktor, der ca. 10 Jahre in Betrieb war und feststoffarme Abwässer der Lebensmitteindustrie mit einem hohen Anteil an Eiweiß und Fett verarbeitete, wurden für die Entschwefelung einzusetzende Mikroorganismen isoliert.

Der Biogasreaktor war zur Mikroorganismenfixierung (Reduktion von Mikroorganismenauswaschung) mit Altreifen gefüllt. Der jahrelange Kontakt der Mikroorganismen im Biogasreaktor mit den freigesetzten Alterungsschutzmitteln und Zinkoxid aus den Altreifen sowie mit dem gebildeten Schwefelwasserstoff im Biogas, bewirkte durch Adaptation an diese biotoxischen Stoffe eine hohe Resistenz der schwefelreduzierenden Bakterien. Positiv wirkte sich auch der Umstand aus, dass die Schwefelreduzierer den polysulfidischen Schwefel an der Oberfläche und im Inneren der sich langsam zersetzenden Altreifen als Substrat verwerten und reduzieren konnten. Bei den isolierten Mikroorganismen handelt es sich um *Sporanaerobacteracetigenes, Desulfovibrio vulgaris, Desulfurella amilsii* und *Desulfomicrobium baculatum.*

### Beispiel 1

Ein Gummimehl aus LKW-Ganzreifen (Gummimehlalter: 2 Jahre), mit einer Korngröße < 0,4 mm wurde mit einer Mischpopulation, die initial gleiche Anteile von *Sporanaerobacter acetigenes, Desulfovibrio vulgaris, Desulfurella amilsii* und *Desulfomicrobium baculatum* enthielt, einer Oberflächenentschwefelung bei folgenden Prozessparametern unterzogen: Temperatur 40 °C, pH-Wert 7, Behandlungsdauer 11 Tage.

In einer ungefüllten Standardgummimischung nach Rezeptur der ASTM D3181 unter Verwendung eines SMR 20 (Naturkautschuk) wurden 15 %(w/w) unbehandeltes Gummimehl (GM 1) und behandeltes (devulkanisiertes) Gummimehl (GM 2) gleichen Anteils in die Standardgummimischung eingearbeitet.

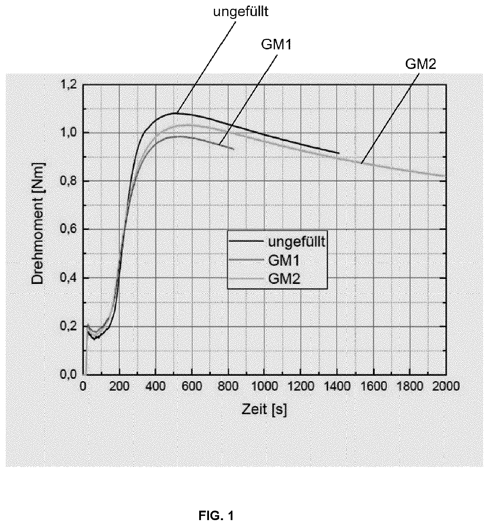

Die drei Gummimischungen wurden einer mechanischen Prüfung im Zugversuch unterzogen (nach DIN 53 mit 200 mm/Minute Prüfgeschwindigkeit, Prüfkörper: Typ 52). Die Vulkanisationsisothermen (bei einer Temperatur von 160 °C) und die Ergebnisse der Zugversuche sind in Figur 1 und Tabelle 1 dargestellt.

**Tabelle 1: Zusammenstellung und Vergleich der Ergebnisse der Zugversuche**

| **Kennwert** | **Einheit** | **Standard** | **GM 1** | **GM 2** |
|---|---|---|---|---|
| δ₅₀ | MPa | 1,38 ± 0,052 | 1,29 ± 0,018 | 1,46 ± 0,011 |
| δ₁₀₀ | | 2,55 ± 0,13 | 2,38 ± 0,038 | 2,72 ± 0,030 |
| δ₂₀₀ | | 6,13 ± 0,35 | 5,75 ± 0,078 | 6,56 ± 0,088 |
| δ₃₀₀ | | 11,4 ± 0,50 | 10,8 ± 0,11 | 12,0 ± 0,11 |
| δ_{M} | MPa | 28,7 ± 0,92 | 20,2 ± 1,01 | 21,7 ± 0,73 |
| ε_{M} | % | 548 ± 7 | 461 ± 16 | 458 ± 10 |
| δ_{B} | MPa | 28,3 ± 0,69 | 20,0 ± 1,2 | 21,5 ± 0,7 |
| ε_{B} | % | 548 ± 7 | 462 ± 15 | 458 ± 10 |

Aus den Ergebnissen ist ersichtlich, dass die Mischung mit dem behandelten Gummimehl (GM 2) Festigkeitswerte (δ₅₀ - δ₃₀₀) aufweist, die im Vergleich zu den korrespondierenden Werten der Gummimischungen mit unbehandeltem Mehl (GM 1) wesentlich besser sind.

Dieser Umstand belegt eindeutig, dass es zu einer kovalenten Anbindung des behandelten Gummimehls an die ungefüllte Standardmischung während der Vernetzungsreaktion gekommen ist. Der Abfall einiger Kennwerte (Zugfestigkeit, Reißfestigkeit) der gefüllten Mischungen gegenüber der ungefüllten Mischung ist teilweise auf das Alter der Gummimehle zurückzuführen.

Zudem muss berücksichtigt werden, dass die höhere Netzwerkdichte der Gummimischung GM 2 (mehr Schwefelbindungen im Vergleich zur Gummimischung GM 1) zwangsläufig zu einem geringen Abfall der Dehnungswerte führt. Dieser Umstand ist für praktische Anwendungsfälle allerdings nicht relevant.

Die Materialtestung erfolgt durch die Polymer Service GmbH Merseburg (www.polymerservice-merseburg.de).

### Beispiel 2

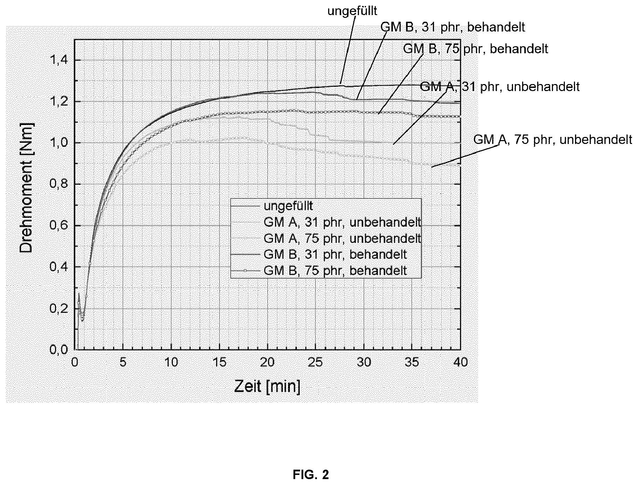

Die Ergebnisse der vergleichenden Werkstoffprüfungen von ungefüllten und gefüllten synthetischen Kautschukmischungen [Ethylen-Propylen-Dien-Mischung (EPDM)] sind in Figur 2 und in Tabelle 2 dargestellt.

**Tabelle 2: Zusammenstellung der Ergebnisse der Zugversuche an EPDM Vulkanisaten**

| **Kennwert** | **Einheit** | **EPDM** | | |
|---|---|---|---|---|
| | | **Standard** | **30 wt-% Gummimehl** | |
| | | | **GM A** | **GM B** |
| | | | **unbehandelt** | **behandelt** |
| δ₅₀ | MPa | 1,53 ± 0,009 | 1,28 ± 0,073 | 1,39 ± 0,008 |
| δ₁₀₀ | | 2,68 ± 0,021 | 2,15 ± 0,013 | 2,38 ± 0,026 |
| δ₂₀₀ | | 5,15 ± 0,037 | 4,12 ± 0,026 | 4,60 ± 0,026 |
| δ₃₀₀ | | 7,67 ± 0,050 | 6,20 ± 0,040 | 6,88 ± 0,005 |
| δ_{M} | MPa | 7,69 ± 1,13 | 7,27 ± 0,172 | 6,80 ± 0,309 |
| ε_{M} | % | 298 ± 42 | 349 ± 7 | 296 ± 13 |
| δ_{B} | MPa | 7,62 ± 1,11 | 7,15 ± 0,197 | 6,76 ± 0,314 |
| ε_{B} | % | 298 ± 42 | 349 ± 7 | 296 ± 13 |

Die Korngröße des Gummimehls betrug ≤ 0,4 mm. Folgende Prozessparameter wurden eingestellt: Prozesstemperatur 40 °C, pH-Wert 7,5, Behandlungsdauer 8 Tage.

Die Ergebnisse der Zugversuche zeigen, dass die für praktische Belange wichtigen Festigkeitswerte (δ₅₀ ... δ₃₀₀) der Gummimischungen mit behandelten (devulkanisierten) Gummimehlen generell oberhalb der Mischung mit dem unbehandelten Gummimehl liegen.

Wegen der unterschiedlichen Vernetzungsdichten liegen die Werte für die ultimativen Eigenschaften (Maximaldehnung ε_{M}, Maximalfestigkeit δ_{M}) für die EPDM mit behandelten Gummimehlen unter denen der Standardmischung und der EPDM mit unbehandeltem Gummimehl.

Aus den Ergebnissen ist ersichtlich, dass auch bei EPDM die devulkanisierten Gummimehle über Schwefelbrücken chemische Bindungen mit ungefüllten (frischen) EPDM eingehen.

## Patentansprüche

1. Verfahren zur Devulkanisation von Gummipartikeln, umfassend die Kultivierung von Bakterien der Gattung *Sporoanaerobacter, Desulfovibrio, Desulfurella* und/oder *Desulfomicrobium* mit Gummipartikeln.

2. Verfahren gemäß Anspruch 1, **dadurch gekennzeichnet, dass** das Verfahren die Kultivierung einer Mischpopulation umfasst, wobei die Mischpopulation *Sporanaerobacter acetigenes, Desulfovibrio vulgaris, Desulfurella amilsii* und *Desulfomicrobium baculatum* umfasst.

3. Verfahren gemäß Anspruch 1 oder 2, **dadurch gekennzeichnet, dass** das Verfahren eine Vorkultur und eine Hauptkultur umfasst.

4. Verfahren gemäß Anspruch 3, **dadurch gekennzeichnet, dass** die Vorkultur eine Mischpopulation von *Sporanaerobacter acetigenes, Desulfovibrio vulgaris, Desulfurella amilsii* und *Desulfomicrobium baculatum* umfasst.

5. Verfahren gemäß Anspruch 3 oder 4, **dadurch gekennzeichnet, dass** als initiales Inokulum der Vorkultur gleiche Anteile von *Sporanaerobacter acetigenes, Desulfovibrio vulgaris, Desulfurella amilsii* und *Desulfomicrobium baculatum* eingesetzt werden.

6. Verfahren gemäß einem der Ansprüche 3 bis 5, **dadurch gekennzeichnet, dass** die Vorkultur Gummimehl umfasst.

7. Verfahren gemäß einem der Ansprüche 1 bis 6, **dadurch gekennzeichnet, dass** das Verfahren im Wesentlichen anaerob ist.

8. Verfahren gemäß einem der Ansprüche 1 bis 6, **dadurch gekennzeichnet dass** das Verfahren das Waschen der Gummipartikel umfasst.

9. Mischpopulation, umfassend Bakterien der Gattung *Sporoanaerobacter, Desulfovibrio, Desulfurella* und *Desulfomicrobium.*

10. Mischpopulation gemäß Anspruch 9, dadurch erhältlich, dass *Sporanaerobacter acetigenes, Desulfovibrio vulgaris, Desulfurella amilsii* und *Desulfomicrobium baculatum* in Anwesenheit von Gummimehl kultiviert werden.

11. Verwendung einer Mischpopulation zur Wiederverwertung von Altgummi durch Devulkanisation, **dadurch gekennzeichnet, dass** die Mischpopulation Bakterien der Gattung *Sporoanaerobacter, Desulfovibrio, Desulfurella* und/oder *Desulfomicrobium* umfasst.

## Claims

1. A process for the devulcanization of rubber particles, comprising culturing bacteria of the species *Sporanaerobacter, Desulfovibrio, Desulfurella* and/or *Desulfomicrobium* with rubber particles.

2. The process according to claim 1, **characterised in that** the process comprises culturing a mixed population, wherein the mixed population comprises *Sporanaerobacter acetigenes, Desulfovibrio vulgaris, Desulfurella amilsii* and *Desulfomicrobium baculatum.*

3. The process according to claim 1 or 2, **characterised in that** the process comprises a preculture and a main culture.

4. The process according to claim 3, **characterised in that** the preculture comprises a mixed population of *Sporanaerobacter acetigenes, Desulfovibrio vulgaris, Desulfurella amilsii* and *Desulfomicrobium baculatum.*

5. The process according to claim 3 or 4, **characterised in that** equal proportions of *Sporanaerobacter acetigenes, Desulfovibrio vulgaris, Desulfurella amilsii* and *Desulfomicrobium baculatum* are used as the initial inoculum of the preculture.

6. The process according to any one of claims 3 to 5, **characterised in that** the preculture comprises rubber flour.

7. The process according to any one of claims 1 to 6, **characterised in that** the process is essentially anaerobic.

8. The process according to any one of claims 1 to 6, **characterised in that** the process comprises washing of the rubber particles.

9. A mixed population comprising bacteria of the species *Sporanaerobacter, Desulfovibrio, Desulfurella* and *Desulfomicrobium.*

10. The mixed population according to claim 9, which can be obtained by *Sporanaerobacter acetigenes, Desulfovibrio vulgaris, Desulfurella amilsii* and *Desulfomicrobium baculatum* being cultivated in the presence of rubber flour.

11. Use of a mixed population for recycling waste rubber by devulcanization, **characterised in that** the mixed population comprises bacteria of the species *Sporanaerobacter, Desulfovibrio, Desulfurella* and/or *Desulfomicrobium.*

## Revendications

1. Procédé de dévulcanisation de particules de caoutchouc, comprenant la culture de bactéries du genre *Sporanaerobacter, Desulfovibrio, Desulfurella* et/ou *Desulfomicrobium* avec des particules d'caoutchouc.

2. Procédé selon la revendication 1, **caractérisé en ce que** le procédé comprend la culture d'une population mixte, ladite population mixte comprenant *Sporanaerobacter acetigenes, Desulfovibrio vulgaris, Desulfurella amilsii* et *Desulfomicrobium baculatum .*

3. Procédé selon la revendication 1 ou 2, **caractérisé en ce que** le procédé comprend une préculture et une culture principale.

4. Procédé selon la revendication 3, **caractérisé en ce que** la préculture comprend une population mixte de *Sporanaerobacter acetigenes, Desulfovibrio vulgaris, Desulfurella amilsii* et *Desulfomicrobium baculatum.*

5. Procédé selon la revendication 3 ou 4, **caractérisé en ce que** des proportions égales de *Sporanaerobacter acetigenes, Desulfovibrio vulgaris, Desulfurella amilsii* et *Desulfomicrobium baculatum* sont utilisées comme inoculum initial de la préculture.

6. Procédé selon l'une des revendications 3 à 5, **caractérisé en ce que** la préculture comprend de la farine de caoutchouc.

7. Procédé selon l'une des revendications 1 à 6, **caractérisé en ce que** le procédé est essentiellement anaérobie.

8. Procédé selon l'une des revendications 1 à 6, **caractérisé en ce que** le procédé comprend le lavage des particules de caoutchouc.

9. Population mixte comprenant des bactéries du genre *Sporanaerobacter, Desulfovibrio, Desulfurella* et *Desulfomicrobium.*

10. Population mixte selon la revendication 9, pouvant être obtenue en cultivant *Sporanaerobacter acetigenes, Desulfovibrio vulgaris, Desulfurella amilsii* et *Desulfomicrobium baculatum* en présence de farine de caoutchouc.

11. Utilisation d'une population mixte pour le recyclage de caoutchouc usagé par dévulcanisation, **caractérisée en ce que** la population mixte comprend des bactéries du genre *Sporanaerobacter, Desulfovibrio, Desulfurella* et/ou *Desulfomicrobium .*
